Europäisches Patentamt

European Patent Office

Office européen des brevets

(11) Publication number: **0 305 968**
**A2**

(12) **EUROPEAN PATENT APPLICATION**

(21) Application number: 88114131.1

(22) Date of filing: 30.08.88

(51) Int. Cl.⁴: **A61K 9/18 , A61K 9/14 ,**
**A61K 47/00 , A61K 31/415 ,**
**A61K 31/285 , A61K 31/47 ,**
**A61K 31/435 , A61K 31/165 ,**
**A61K 31/42 , A61K 31/255 ,**
**A61K 31/54**

(30) Priority: 31.08.87 IL 83715

(43) Date of publication of application:
08.03.89 Bulletin 89/10

(84) Designated Contracting States:
AT BE CH DE ES FR GB GR IT LI LU NL SE

(71) Applicant: **YEDA RESEARCH AND**
**DEVELOPMENT COMPANY, LIMITED**
**P.O. Box 95**
**Rehovot 76100(IL)**

(72) Inventor: **Mirelman, David**
**5 Harduf Street**
**Ramat Efal(IL)**
Inventor: **Wilchek, Meir**
**3 Haavoda Street**
**Rehovot(IL)**

(74) Representative: **Vossius & Partner**
**Siebertstrasse 4 P.O. Box 86 07 67**
**D-8000 München 86(DE)**

(54) Compositions against protozoal diseases.

(57) The invention relates to pharmaceutical compositions for use against protozoa. The compositions comprise particles in the micron-size range which are physiologically acceptable to humans and which can be ingested by protozoa. The particles, which can be of natural materials such as silica or the like, serve as carrirs of an effective anti-protozoal drug. A variety of anti-amebic drugs can be bound to such particles.

Fig. 1

EP 0 305 968 A2

## Compositions against Protozoal Diseases

Field of the Invention:

It is the object of the invention to provide anti-protozoal agents having a high degree of activity and a reduced adverse effect on patients treated with such drugs. The pharmaceutical compositions of the invention comprise an antiprotozoal agent bound to suitable inert small particles, adapted to be ingested by the protozoa. The carrier-bound drug is advantageously provided in the form of particles of a size adapted to be ingested by protozoa.

Background of the Invention:

Entamoeba histolytica is an intestinal protozoan parasite causing amebic dysentery, an invasive disease which can also sometimes produce liver or brain abscesses which may be fatal. The disease is very prevalent in countries with bad sanitary conditions and where potable waters may be contaminated with fecal material. Over 400 million people in the world harbor and carry such parasites, although only 10% of these ever show clinical symptoms. Recent information suggests that amebae in asymptomatic carriers may also have a pathogenic potential and that these carriers should be treated to prevent an unexpected outburst, and to eliminate the source of infection.

A large number of drugs are used against amebae. Of these, the most commonly used are derivatives of nitroimidazoles (Tables 1,2 and 3). Although the exact mechanisms of action of these drugs are not completely clear, they are believed to involve the reduction of the nitro-group by the anaerobic systems of the parasite, most likely by ferredoxin molecules, which produces an active radical that blocks protein and nucleic acid synthesis. The nitroimidazoles are very efficient growth inhibitors of E. histolytica and their $LD_{50}$ in vitro is approximately 1 μg/ml. Nitroimidazoles are very efficient drugs, especially in cases of extraintestinal lesions because they are well absorbed in the small intestine. Nitroimidazoles cause sideeffects in many patients, and they kill indiscriminately the anaerobic bacteria which constitute most of the normal intestinal flora. They are suspected of having a carcinogenic potential since they have been shown to induce mutagenesis in bacteria (Ames test). Nitroimidazoles have also been shown to have a weak effect in eliminating amebae from the lumen of large intestines of asymptomatic carriers, probably because there is not attained a significant luminal concentration.

Entamoeba histolytica can nowadays be easily cultivated in vitro. Trophozoites are large phagocytic cells (mean size 30-50 μ diameter), which readily ingest certain species of bacteria and a variety of nutrient particles such as starch granules and red blood cells. Phagocytosis is mediated by recognition mechanism between molecules on the surface of the trophozoites and receptors on the ingested cell or particle. Following ingestion, amebic enzymes cause the breakdown and digestion of the particles. Trophozoites also ingest a variety of inert particles such as silica, alumina silicate and latex beads.

Summary of the Invention:

The invention relates to carrier-bound antiprotozoal drugs. Specifically, there are provided anti-amebic drugs bound to inert carrier particles, adapted to be ingested by the protozoa.

Although applicable to a wide variety of protozoa, especially intracellular parasites, the present invention is illustrated with reference to ameba.

The particulate drug is in such form that it will be readily ingested by the phagocytic system of the protozoa, and more specifically by Entamoeba histolytica.

As the size of amebae is of the order of about 50 μ, the particulate drug is provided in the form of particles in the range of about 0.1 μ to about 10 μ, and preferably about 1 μ to about 5 μ particle size.

There may be used a wide range of biocompatible inert physically acceptable particles. The active drug is bound to such particles, preferably by a chemical bond, and such particles are ingested by protozoa, the active drug is released to exert its anti-amebic effect. The absorption of the drug from the intestine is greatly decreased, and thus side-effects are minimized. Also the effect on the intestinal flora of the patient is greatly reduced.

The particle bound drug will be readily available to the amebic trophozoites present in the intestinal

2

lumen, thus providing an efficient means of treatment of asymptomatic carriers. It is apparent that the overall dosage of the effective ingredient can be substantially reduced as the drug is effectively targeted to the protozoae.

A suitable form can be particles where the drug may be chemically bound to latex beads, or to inorganic particles such as silicas, aluminum-silicates (kaolin), or other particles which contain silicon dioxide (SiO$_2$) that are also well taken up by amebic trophozoites The preferred particle size is in the 2-10 micron range.

The binding of the drug to the carrier can be effected by conventional chemical means. The binding can be via functional groups such as hydroxy groups, amino groups, or the like. The carrier particles can be chemically modified by coupling them with functional molecules which contain aldehydes, carboxyls, hydroxyls, epoxy or amino groups, or the like. The quantity of active drug bound to the carrier varies, it is generally in the range of about 10 to 250 mg/g of dry particle.

A wide range of suitable antiprotozoal drugs is set out in the enclosed Tables 1, 2 and 3; these are suitable for linkage to such particulate carriers.

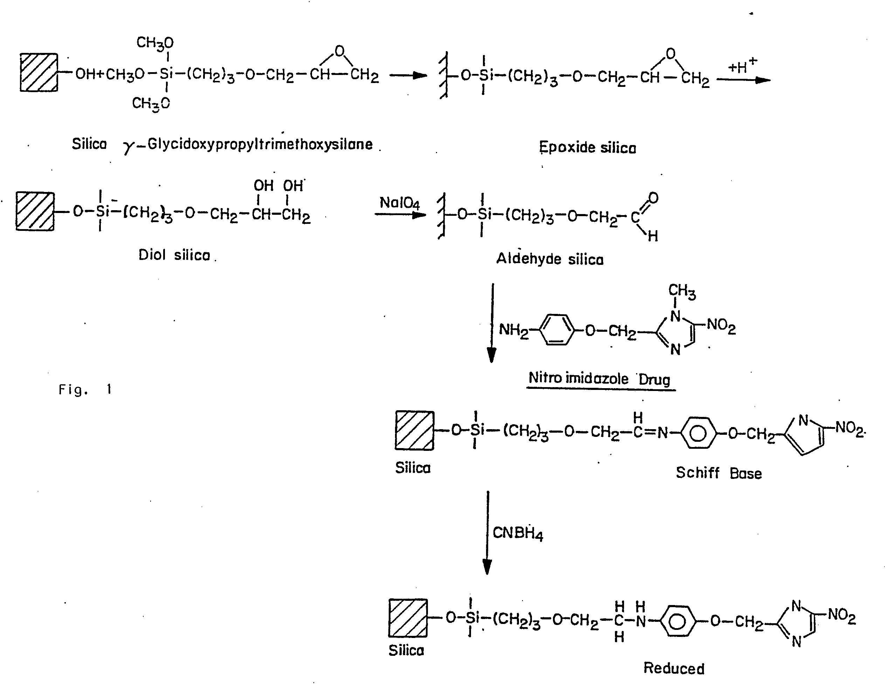

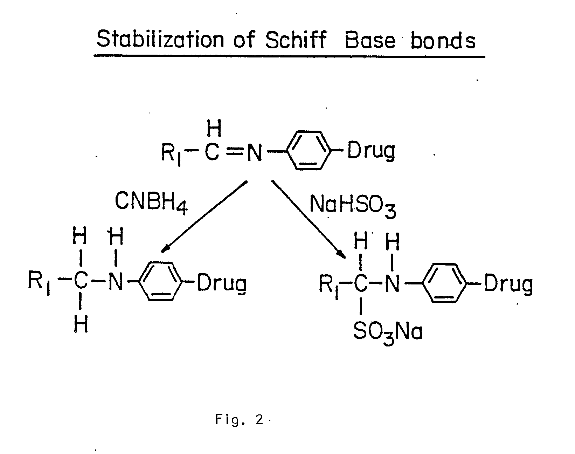

A Nitroimidazole derivative which contains an aromatic amino group (Hoechst AG # S 74 8663) that has very similar activity on E. histolytica as Metronidazole (FLAGYL®), was covalently linked with particles containing aldehyde groups on their surface. The particles were diol subsituted silica or aluminum- silicate particles Fig. 1), in which aldehyde groups can be formed by pretreatment with periodate that oxidizes vicinal hydroxyl groups and yields aldehyde groups. Interaction of such aldehyde substituted particles with compounds containing free amino groups leads to the formation of Schiff bases, resulting in morphologically similar particles which possess a yellowish color. Schiff base was then interacted with sodium bisulfite to stabilize the bond (Fig. 2). The amount of drug which can be optimally bound to the beads is about 10 - 100 μg/mg dry particle. One mg of dry weight silica particles contains 10$^7$-10$^9$ particles depending on the source.

Silica beads conjugated to nitroimidazole derivatives added to cultures of Entamoeba histolytica were rapdily ingested by trophozoites (>5-10 particles/cell in 30 minutes) and were extremely efficient at killing the amebae. Within 1 h most trophozoites were inactivated and killed. The minimal dose as tested so far was 3-14 μg/ml and ingestion was 2-10 particles/ameba. Controls containing only silica beads which had been pretreated with periodate and interacted with amino acids and sodium bisulfite did not cause any damage to the amebae. Amebae treated with Metronidazole alone required 1-5 μg/ml and over 24 h to kill more than 95% of the trophozoites.

Reduction with borohydride of the Schiff base formed between the amino groups of the drug and the aldehyde group of the carrier particle yielded a stable, covalently bound drug (Fig. 2). Amebae treated with this drug were very poorly inactivated.

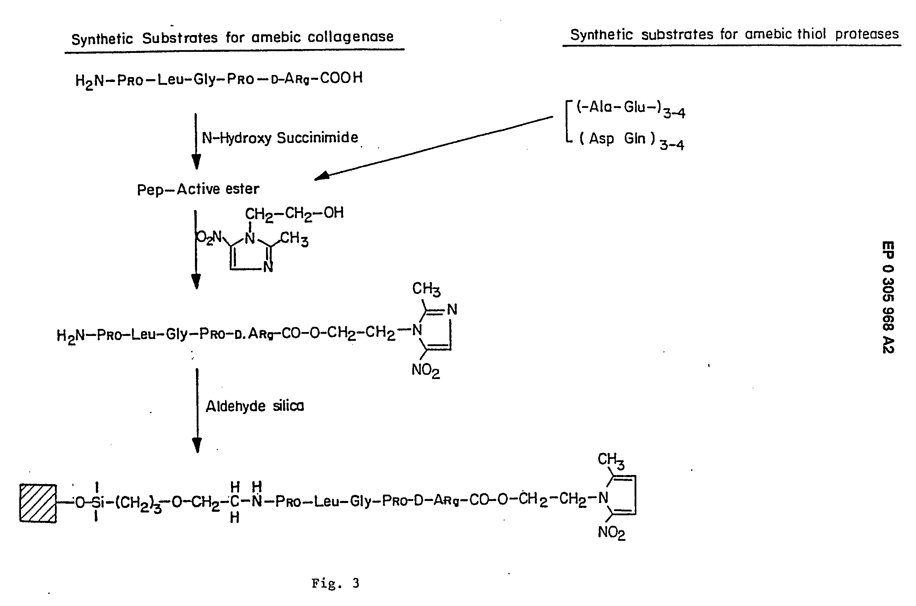

Silica beads were chemically stable and not digested by the amebae enzymes. Unbound nitroimidazole solutions, given orally or placed in the small intestine of mice, could be detected in the serum as early as 1 h after administration. Serum samples of animals in which silica conjugated-drug was placed in the intestine did not contain any drug. In order to facilitate the release of the drugs bound to the silica particle by the amebic enzymatic systems the drugs were also covalently linked to collagen like synthetic peptides (4-8 amino acids), or peptides by means of ester or peptide bonds which are cleaved by thiolproteases or esterases of the amebae, and their peptide drug conjugates were then linked to the aldehyde silica beads and reduced with sodium borohydride to stabilize the bond (Fig. 3).

Silica particles are known to be toxic to mammalian macrophages but silica dioxide was declared safe as a food additive by FAO/WHO.

Other antiamebic drugs were tested using the same principle. These include various derivatives of nitroimidazoles, quinacrine, colistin, diloxanide furoate, paromomycin, ethidium bromide, and more recently allicin, the active ingredient of garlic - all of which (or their derivatives) can be covalently bound to silica particles.

The principle of binding a drug to an inert carrier particle which will be specifically taken up by the phagocytic system of amebae is the basic conept of our invention. Various possible chemical routes to covalently link an antiamebic drug to different types of particles can be used. Aldehyde-amino group condensation is only one of them, and others can also be used. The advantages of our invention are that the conjugated drug significantly decreases the side effects of the drug, and that the drug will not adversely affect the bacterial intestinal anaerobic flora. The conjugated drug is available to the trophozoites present in the intestinal lumen, which is the case in the majority of human parasite carriers. Drug doses can be greatly reduced and the drug-conjugate can be given prophylactically.

The drug can also be conjugated to suitable carrier particles such as aluminosilicates (kaolin).

3

Table 1: 4-Nitro imidazoles

| Comp. Name | $R_1$ | $R_2$ |
|---|---|---|
| Fexinidazole(HOE 239) | $-Ch_2O-\langle o \rangle-SCH_3$ | $-CH_3$ |
| Go.10213 | (2-oxo-imidazolidinyl)-N-$SO_2CH_3$ | $-CH_3$ |
| HOE 239(Sulphoxide) | $-CH_2O-\langle o \rangle-SCH_3$ | $-CH_3$ |
| HOE 339(Sulphone) | $-CH_2O-\langle o \rangle-SO_2CH_3$ | $-CH_3$ |
| Nitrimidazine | $-H$ | $-CH_2CH_2-N\langle morpholino \rangle O$ |

Table 2: 5-Nitrol imidazoles

| Comp. Name | $R_1$ | $R_2$ |
|---|---|---|
| Carnidazole | $-CH_2CH_2NHCSOCH_3$ | $-CH_3$ |
| Dimetridazole | $-CH_3$ | $-CH_3$ |
| Flunidazole | $-CH_2CH_2OH$ | $-\langle\bigcirc\rangle-F$ |
| Metronidazole | $-CH_2CH_2OH$ | $-CH_3$ |
| Nimorazole | $-CH_2CH_2-N\langle\quad\rangle O$ | $-H$ |
| Ornidazole | $-CH_2CH(OH)CH_2Cl$ | $-CH_3$ |
| Panidazole | $-CH_2CH_2-\langle\bigcirc\rangle N$ | $-CH_3$ |
| Ronidazole | $-CH_3$ | $-CH_2OCONH_2$ |
| Satranidazole | $-CH_3$ | $-N\langle\quad\rangle N-SO_2CH_3$ , $=O$ |
| SC-28538 | $-CH_3$ | $-SCH_2CH_2O-\langle\bigcirc\rangle-COO$ |
| Secnidazole | $-CH_2CH(OH)CH_3$ | |
| S750400A | $-CH_3$ | $-CH_2O-\langle\bigcirc\rangle-NCHN(CH_3)_2$ |
| Tinidazole | $-CH_2CH_2SO_2CH_2CH_3$ | $-CH_3$ |

5

Table 3: 2-Nitro imidazoles

| Comp. Name | $R_1$ | $R_2$ |
|---|---|---|
| Benznidazole | $-CH_2CONHCH_3$ | $-H$ |
| L-10333 | $-CH_3$ | $-COOCH_3$ |
| L-6678 | $-CH_2CH_2OH$ | $-CH_3$ |
| L-8580 | $-CH_3$ | $-CH{=}CH_2$ |
| L-8711 | $-CH_3$ | $-CHO$ |
| Misonidazole | $-CH_2CH(OH)CH_2OCH_3$ | $-H$ |
| RGW-613 | $-CH_2COOCH_3$ | $-H$ |
| RGW-801 | $-CH_3$ | $-CN$ |
| RGW-806 | $-CH_3$ | $-CONH_2$ |

Table 4: Additional drugs which can be chemically linked to silica particles

Allicin
Amidiaquine
Bemarsal
Chloroquine
Colistin
Dehydroemetine
Diiodohydroxyquinoline
Diloxanide furoate
Emetine dihydrochloride
Enterovioform
Ethidium bromide
Furazolidone
Imidocarb
Niridazole
Nifurtimox
Nitrofurantoin
Paramoxycin
Quinacrine dihydrochloride
4-aminoquinoline
8-hydroxyquinoline
Sulfasalazine
Sulfapyrazine

6

**Claims**

1. An antiprotozoal pharmaceutical composition comprising micron-size particles of physiologically acceptable natural particles selected from silica, aluminum silicates, kaolin, latex etc., which are ingestable by protozoa, and an anti-protozoal drug being bonded to said particles.

2. A composition according to claim 1, where the carrier is selected from inert minerals such as silica, aluminum-silicate or from latex beads or the like.

3. A composition according to claim 1 or 2, where the carrier particles are of a size in the range of from 0.1 μ to about 10 μ.

4. A composition according to any of claims 1 to 3, where the drug is chemically bonded to the carrier.

5. A composition according to any of claims 1 to 4, where the drug is an anti-amebic drug selected from 5-nitroimidazole type anti-amebics.

6. A composition according to any of claims 1 to 4, where the drug is a 2-nitro-, a 4-nitro- or a 5-nitro-imidazole anti-amebic drug.

7. A composition according to claim 6, where the drug is one set out in any of the Tables of nitroimidazole drugs presented herein.

8. A composition according to any of claims 1 to 4, where the drug is selected from amidiaquine, bemarsal, chloroquine, colistin, diiodohydroxyquinoline, diloxanide furoate, emetine dihydrochloride, enterovioform, ethidium bromide, furazolidone, imidocarb, allicin, oxyquinoline, nifurtimox, nitrofurantoin, paramoxycin, quinacrine dihydrochloride, 4-aminoquinoline, 8-hydroxyquinoline and niridazole.

9. A composition according to any of claims 1 to 8, where the binding is by a chemical bond via a hydroxy, aldehyde or amino group.

10. A composition according to any of claims 1 to 8, where the carrier consists of silica, aluminum-silicate, kaolin, latex beads or similar particles in the range of 0.1 μ to about 10μ size to which there is bonded from 30 mg to about 250 mg anti-amebic drug per gram of carrier.

11. A composition according to claim 1, where the bonding is by adsorbtion on suitable carrier particles.

12. Compositions according to any of claims 1 to 10, where the drug is first linked to a peptide or collagen link and such conjugate is subsequently bonded to the particles.

13. Use of micron-size particles selected from silicon, aluminium silicates, kaolin, latex, or the like for the preparation of an anti-protozoal pharmaceutical composition by bonding to said particles an anti-protozoal drug which may have optionally been linked to a peptide or collagen link forming a conjugate prior to be bonded to the particles.

Fig. 1

13

# Stabilization of Schiff Base bonds

Fig. 2

EP 0 305 968 A2

Synthetic Substrates for amebic collagenase

Synthetic substrates for amebic thiol proteases

$H_2N-Pro-Leu-Gly-Pro-D-Arg-COOH$

$\begin{bmatrix} (-Ala-Glu-)_{3-4} \\ (Asp\ Gln)_{3-4} \end{bmatrix}$

$\downarrow$ N-Hydroxy Succinimide

Pep—Active ester

$\downarrow$

$H_2N-Pro-Leu-Gly-Pro-D.Arg-CO-O-CH_2-CH_2-N$ ... $CH_3$, $N$, $NO_2$

$\downarrow$ Aldehyde silica

—O—Si—$(CH_2)_3$—O—$CH_2$—C—N—Pro—Leu—Gly—Pro—D—Arg—CO—O—$CH_2$—$CH_2$—N ... $CH_3$, $NO_2$

Fig. 3

EP 0 305 968 A2